Europäisches Patentamt

European Patent Office

Office européen des brevets

(19)

(11) Veröffentlichungsnummer: **0 217 034**
**B1**

(12) ## EUROPÄISCHE PATENTSCHRIFT

(45) Veröffentlichungstag der Patentschrift:
**31.01.90**

(51) Int. Cl.⁴: **A61F 2/36**

(21) Anmeldenummer: **86110037.8**

(22) Anmeldetag: **22.07.86**

(54) Geradschaft aus Metall für eine Femurkopfprothese.

(30) Priorität: **30.08.85 CH 3732/85**

(43) Veröffentlichungstag der Anmeldung:
**08.04.87 Patentblatt 87/15**

(45) Bekanntmachung des Hinweises auf die Patenterteilung:
**31.01.90 Patentblatt 90/5**

(84) Benannte Vertragsstaaten:
**AT DE FR GB IT**

(56) Entgegenhaltungen:
**EP-A- 0 085 147**
**EP-A- 0 131 178**
**EP-A- 0 149 527**
**EP-A- 0 196 258**
**EP-A- 0 201 442**
**FR-A- 1 158 993**
**FR-A- 2 519 248**
**GB-A- 2 059 267**
**US-A- 3 685 058**
**US-A- 3 918 441**

(73) Patentinhaber: **GEBRÜDER SULZER AKTIENGESELLSCHAFT, Zürcherstrasse 9, CH-8401 Winterthur(CH)**

(72) Erfinder: **Griss, Peter, Prof. Dr.-med. Orthopädische Klinik, im Klinikum der Philipps-Universität, Baldingerstrasse D-3550 Marburg(DE)**

(74) Vertreter: **Sparing Röhl Henseler Patentanwälte European Patent Attorneys, Rethelstrasse 123, D-4000 Düsseldorf 1(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft einen blattartigen Geradschaft aus Metall für eine Femurkopfprothese.

Werden blattartige Geradschäfte zementfrei im Femur verankert, so haben sich in der Praxis vor allem zwei Mängel gezeigt. Bekanntlich ist bei einer zementfreien Verankerung die Primärfixation des Implantates ein grundlegendes Problem, das im allgemeinen durch ein Verklemmen des Schaftes im ausgeräumten Femurknochen gelöst wird. Dabei sind häufig – bei gleichen Schaftlängen und -breiten, d.h. gleichen Abmessungen proximal/distal und medial/lateral – unterschiedliche Dicken des Geradschaftes, d.h. unterschiedliche Dimensionen in Richtung anterior/posterior erforderlich, um einen optimalen Klemmeffekt zu erzielen. Diese Forderung vergrößert die Anzahl Schäfte, die sich nur in der genannten Dimension unterscheiden, beträchtlich. Darüberhinaus ist häufig erst intraoperativ zu ermitteln, welche Schaftdicken im Einzelfall benötigt werden, weshalb mehrere Schäfte unterschiedlicher Dicke unter sterilen Bedingungen bereitgestellt werden müssen.

Das zweite Problem ist technischer Natur und besteht darin, daß sich besonders bei Schäften, deren Querschnitt von der einfachen Rechteckform abweicht, für das An- oder Aufbringen der Oberflächenstruktur aufwendige und relativ komplizierte Arbeitsprozesse notwendig sind.

Aus der EP-A 0 196 258, die nach Art. 54(3) EPÜ den einschlägigen Stand der Technik darstellt, ist ein Schaft bekannt, dessen Kern im proximalen Bereich zur Mittelebene des Schaftes parallele Auflageflächen für Formstücke besitzt. Diese sind in einer Führungsnut geführt; es gibt dort jedoch weder einen Anschlag für eine Begrenzung der Einschiebbewegung der Formstücke nach distal, noch sind Sicherungen gegen ein unbeabsichtigtes Ausschieben der Formstücke vorgesehen.

Aufgabe der Erfindung ist es, einen Prothesenschaft zu schaffen, bei dem ebenfalls eine intraoperative Änderung der Schaftdicke möglich und die Herstellung einfach sind.

Diese aus zwei unabhängigen Teilaufgaben bestehende Doppelaufgabe wird mit einem blattartigen Geradschaft gelöst, der in seinem proximalen Bereich eine Verankerungsstruktur zum An- und Einwachsen von Knochengewebe aufweist, welcher Metallschaft in seinen Blattseiten über die vertikale Höhe des strukturierten proximalen Bereiches mit einer Führungsnut versehene Ausnehmungen hat, die mit die Schaftform und die Oberflächenstruktur verkörpernden separaten Formstücken gefüllt sind, wobei Sicherungen gegen ein unbeabsichtigtes Ausschieben der Formstücke aus der Führungsnut vorgesehen sind.

Das Ausfüllen der Ausnehmungen mit den separaten Formstücken kann dabei vor oder nach dem Einsetzen des Schaftes in den Knochen erfolgen, wobei der Operateur Formstücke unterschiedlicher Dicke versuchsweise verwenden und die geeignete Dicke für einen optimalen Klemmeffekt – der einen festen Sitz gewährleistet, jedoch den Knochen nicht zu stark belastet – auswählen kann. Die Fabrikation wird durch separate Formstücke erheblich vereinfacht, da die Bearbeitung für die Herstellung der Verankerungsstruktur an den geometrisch einfachen Formstücken erfolgt.

Besonders einfach wird die Bearbeitung, wenn die Formstücke aus Kunststoff bestehen und die Verankerungsstruktur durch ein Metallgitter, beispielsweise ein Drahtnetz, gebildet wird. Dieses kann durch einfache Verformung an die, die Schaftform verkörpernde Aussenfläche angepasst und durch teilweises Einpressen in den durch Temperaturerhöhung erweichten Kunststoff fixiert werden. Das Einsetzen der Formstücke in die Ausnehmungen des Schaftes kann erleichtert werden, wenn die Ausnehmungen in einem distalen Anschlag für die Formstücke enden und/oder wenn die Formstücke mit Befestigungsmitteln für ein Führungs- und Einsetzwerkzeug versehen sind.

Wegen der Krümmungen des Femurknochens kann es vorkommen, dass der Schaft nicht zentrisch, sondern versetzt in der Operationsöffnung angeordnet ist. In diesem Fall ist es für einen optimalen Klemmeffekt vorteilhaft, wenn das Formstück für die eine Blattseite eine grössere Dicke aufweist als dasjenige für die andere Seite.

Bevorzugte Materialien für die Metallteile sind Titan oder Titanlegierungen, während Kunststoffteilstücke in erster Linie aus Polyäthylen der in der Implantat-Technik üblichen Modifikationen hergestellt werden.

Im folgenden wird die Erfindung anhand eines Ausführungsbeispiels im Zusammenhang mit der Zeichnung näher erläutert.

Fig. 1 zeigt eine Aufsicht auf eine Ausnehmung im proximalen Bereich einer Blattseite eines Geradschaftes;

Fig. 2 gibt ein zum Schaft von Fig. 1 passendes Formstück, wobei die Aufsicht auf die "Rückseite", d.h. die bei eingesetztem Formstück unsichtbare, dem Schaft zugewandte Seite, dargestellt ist;

Fig. 3 ist die gleiche Darstellung wie Fig. 1 eines Schaftes mit eingesetztem Formstück;

Fig. 4 ist der Schnitt IV-IV von Fig. 3;

Fig. 5 schliesslich ist eine Ansicht des Schaftes von lateral, d.h. eine Ansicht von Fig. 3 von links.

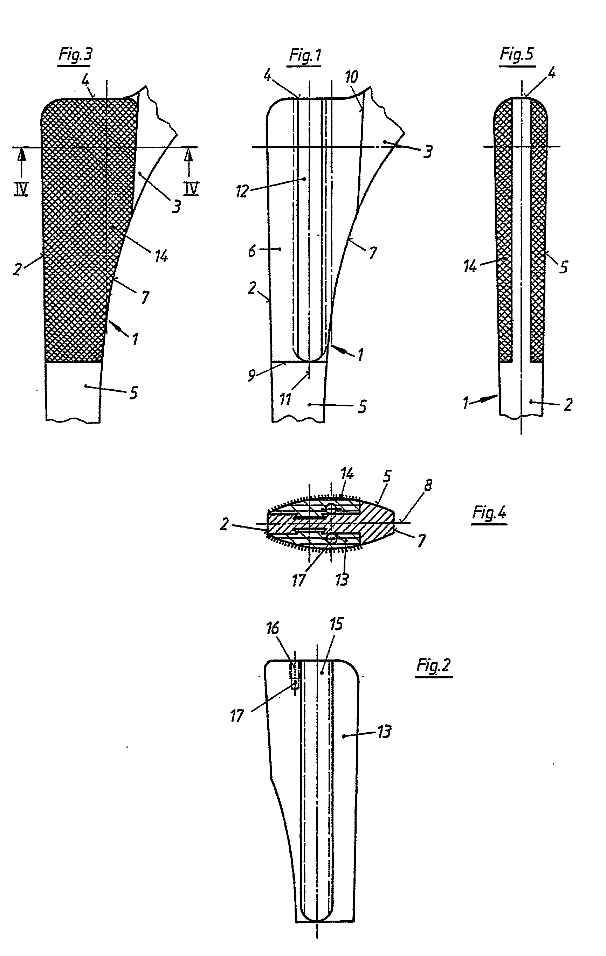

Der sich nach distal verjüngende Geradschaft 1 (Fig. 1), dessen laterale Schmalseite 2 sich an seinem proximalen Ende über eine horizontale Schulter 4 in den nur in seinem Ansatz angedeuteten Prothesenhals 3 fortsetzt, hat in beiden Blattseiten 5 (Fig. 5) sich über die Höhe seines proximalen Bereiches erstreckende Ausnehmungen 6. Der Schaft 1, dessen mediale Schmalseite 7 von distal in einem Bogen in den Prothesenhals 3 übergeht, ist im Querschnitt linsenförmig (Fig. 4), d.h. mit einer bombierten äusseren Oberfläche versehen.

Die Ausnehmungen 6 sind dagegen durch ebene Bodenflächen begrenzt, die parallel zu einer Mittelebene 8 (Fig. 4) verlaufen, die ihrerseits die Symmetrieebene für den Schaftquerschnitt bildet.

Nach distal endet die Ausnehmung 6 in einem Anschlag oder Absatz 9, während der Uebergang in den Prothesenhals 3 in einer Kante 10 besteht, die in Richtung proximal/distal leicht gegen die Längsachse 11 geneigt verläuft. Im Boden der Ausnehmung 6 ist eine schwalbenschwanzförmige Führungsnut 12 vorgesehen, die parallel zur Achse 11 angeordnet ist.

Als Füllung für die Ausnehmungen 6 dienen Formstücke 13, die medial und lateral an die Schaftform angepasst sind und eine den linsenförmigen Querschnitt des Gesamtschaftes gewährleistende bombierte Aussenfläche (Fig. 4) haben. Sie können aus Kunststoff oder aus Metall gefertigt sein und sind auf ihren Aussenflächen mit einer Oberflächenstruktur versehen, die beispielsweise aus einem ein- oder mehrlagigen Metallgitter oder Drahtnetz 14 besteht. Für ihre Verankerung in der Führungsnut 12 haben sie einen entsprechend vorspringenden "Schwalbenschwanz" 15. Weiterhin ist in ihre horizontale Schulter eine mit einem Gewinde 16 versehene Bohrung 17 eingebracht, in der ein Setzinstrument lösbar befestigt werden kann. Gegen ein unbeabsichtigtes Ausschieben aus der Führungsnut 12 ist das Formstück 13 beispielsweise durch nicht dargestellte Schnappvorrichtungen gesichert.

Durch entsprechend geformte andere Formstücke 13 können selbstverständlich auch andere Querschnittsformen des Schaftes 1 - z.B. Rechteckquerschnitte - und/oder andere Oberflächenstrukturen verwirklicht werden, an die die Form der Blattseiten 5 am medialen Uebergang in den Prothesenhals 3 bzw. am distalen Absatz 9 entsprechend angepasst werden. Weiterhin können die Formstücke 13 auch - besonders bei im wesentlichen rechteckigen Querschnitten - gegenüber den Absätzen 9 und den Kanten 10 hervorstehen oder zurückspringen, falls für einen optimalen Klemmsitz eine grössere oder kleinere Dicke erforderlich ist. In diesem Zusammenhang ist es auch möglich, falls notwendig, in eine Blattseite 5 ein dickes und in die andere ein dünnes Formstück 13 einzusetzen.

## Patentansprüche

1. Blattartiger Geradschaft (1) aus Metall für eine Femurkopfprothese, der in seinem proximalen Bereich eine Verankerungsstruktur (14) zum An- und Einwachsen von Knochengewebe aufweist, welcher Metallschaft (1) in seinen Blattseiten (5) über die vertikale Höhe des strukturierten proximalen Bereiches mit einer Führungsnut (12) versehene Ausnehmungen (6) hat, die mit die Schaftform und die Oberflächenstruktur verkörpernden separaten Formstücken (13) gefüllt sind, wobei Sicherungen gegen ein unbeabsichtigtes Ausschieben der Formstücke (13) aus der Führungsnut (12) vorgesehen sind.

2. Geradschaft nach Anspruch 1, dadurch gekennzeichnet, daß die Ausnehmungen (6) in einem distalen Anschlag (9) für die Formstücke (13) enden.

3. Geradschaft nach Anspruch 1, dadurch gekennzeichnet, daß die Oberflächenstruktur durch ein auf das Formstück (13) aufgebrachtes Metallgitter, insbesondere ein Drahtnetz (14), gebildet ist.

4. Geradschaft nach Anspruch 1, dadurch gekennzeichnet, daß die Formstücke (13) mit Befestigungsmitteln (16, 17) für ein Führungs- und Einsetzwerkzeug versehen sind.

5. Geradschaft nach Anspruch 1, dadurch gekennzeichnet, daß das Formstück (13) für die eine Blattseite eine größere Dicke aufweist als dasjenige für die andere Seite.

6. Geradschaft nach Anspruch 1, dadurch gekennzeichnet, daß die Formstücke (13) aus Kunststoff, insbesondere aus Polyäthylen, bestehen.

## Claims

1. A straight blade-like metal stem (1) for a femoral head prosthesis, the stem having in its proximal zone a fixing structure (14) for the ongrowth and invasion of bone tissue, the blade sides (5) of the stem (1) being formed over the vertical height of the structured proximal zone with recesses (6) in which a guide groove (12) is present and which are filled with separate mouldings (13) making up the stem shape and the surface structure, means being provided to prevent accidental removal of the mouldings (13) from the guide groove (12).

2. A stem according to claim 1, characterised in that the recesses (6) terminate in a distal abutment (9) for the mouldings (13).

3. A stem according to claim 1, characterised in that the surface structure is embodied by a metal mesh, more particularly a wire mesh (14), placed on the moulding (13).

4. A stem according to claim 1, characterised in that the mouldings (13) have securing means (16, 17) for a guiding and fitting tool.

5. A stem according to claim 1, characterised in that the moulding (13) has a greater thickness for one blade side than for the other side.

6. A stem according to claim 1, characterised in that the mouldings (13) are made of plastics, more particularly polyethylene.

## Revendications

1. Tige rectiligne (1) en métal du type spatule, pour une prothèse de tête de fémur, présentant, dans sa région proximale, une structure d'ancrage (14) destinée à la croissance périphérique et interne de tissu osseux, cette tige métallique (1) comportant, dans les côtés (5) de sa spatule, des évidements (6) qui sont munis d'une rainure de guidage (12) sur la hauteur verticale de la région proximale structurée et sont comblés par des pièces moulées distinctes (13) matérialisant la forme de la tige et la structure superficielle, des systèmes d'arrêt étant prévus pour empêcher les pièces moulées (13) de se déboîter involontairement de la rainure de guidage (12).

2. Tige rectiligne selon la revendication 1, caractérisée par le fait que les évidements (6) s'achèvent par une butée distale (9) destinée aux pièces moulées (13).

3. Tige rectiligne selon la revendication 1, caractérisée par le fait que la structure superficielle est constituée d'un entrelacs métallique implanté sur la pièce moulée (13), notamment d'un réseau (14) de fils métalliques.

4. Tige rectiligne selon la revendication 1, caractérisée par le fait que les pièces moulées (13) sont pourvues de moyens (16, 17) de fixation d'un instrument de guidage et d'insertion.

5. Tige rectiligne selon la revendication 1, caractérisée par le fait que la pièce moulée (13) destinée à l'un des côtés de la spatule présente une plus grande épaisseur que celle destinée à l'autre côté.

6. Tige rectiligne selon la revendication 1, caractérisée par le fait que les pièces moulées (13) consistent en une matière plastique, notamment en du polyéthylène.

EP 0 217 034 B1